# EUROPEAN PATENT APPLICATION

(11) **EP 3 808 176 A1**
(43) Date of publication of application: **21.04.2021**
(21) Application number: 19808151.5
(22) Date of filing: 24.05.2019
(51) Int. Cl.: A01N 1/02, C12N 5/07, C12N 5/071

(54) **METHOD FOR SUPPRESSING FREEZING DAMAGE AND COMPOSITION FOR PREVENTING FREEZING DAMAGE**

(30) Priority: 25.05.2018 JP 2018100759
(71) Applicant: Kyoto University, Kyoto-shi, Kyoto 606-8501 (JP)
(72) Inventor: HAGIWARA Masatoshi, Kyoto 606-8501 (JP); KANEMITSU Eisho, Kyoto 606-8501 (JP); TANAKA Nobuo, Kyoto 606-8501 (JP)
(74) Representative: Dehns
(86) International application number: PCT/JP2019/020757
(87) International publication number: WO 2019/225753

(57) **Abstract**

Provided is a method capable of suppressing cold damage to a biological material such as an organ even if the biological material is preserved at low temperature for a long time, or a composition for preventing cold damage to a biological material.

Provided also is a method for suppressing cold damage to a biological material, including inhibiting a sphingosine-1-phosphoric acid (S1P)-mediated signal transduction pathway in the biological material; and a cold damage prevention composition containing, as an active ingredient, a substance that inhibits S1P-mediated signal transduction pathway.

## Description

### Technical Field

The present disclosure relates to a method for suppressing damage to a biological material caused by cold storage, a composition for preventing cold damage, a method for preserving a biological material at low temperature, and a solution for preserving a biological material.

### Background Art

There are considered to be many patients who have no means other than organ transplantation for improving prognosis, suffering from decompensated organ failures of the heart, the lung, the liver, the pancreas, the kidney, and the small intestine, or malignancies in these.

In North America, where the transplantation therapy is most often used, 50,000 registrants wait for organ transplantation every year, but less than 10 % of them can receive the transplantation therapy actually. Besides, it is said that 700,000 or more patients die from decompensated organ damage every year, from which it is inferred that there are potential transplant adapters in the patients who die before registering for organ transplantation.

There is no doubt that the greatest cause of the donor shortage is the absolute shortage of brain-dead donors. However, when selecting a donor, access time to an organ is also taken into consideration in addition to histocompatibility, and therefore, if the access time exceeds the upper limit of the storage time, the organ could be discarded due to deterioration of the organ conditions, etc., even though there is a matching recipient with high priority. In light of this, there is no doubt that extending the storage time is an effective and efficient measure against donor shortage. Particularly in the cases where the removed organ is the heart or the lung, which can be preserved for only several hours, the discard rate of the removed organs exceeds 70 %.

Research on the organ preservation is being conducted in each country. The standard method, however, has been unchanged for more than 20 years, which is cold storage of an organ with use of an organ protection solution (Patent Documents 1 and 2), and with this method, the time for which each organ can withstand cold storage is the upper limit of the storage time.

### Prior Art Document

### Patent Document

Patent Document 1: JP-A-2010-239963
Patent Document 2: JP-T-2010-529053

### SUMMARY OF THE INVENTION

### Problem to be Solved by the Invention

With increased time for which an organ removed for organ transplantation can be preserved, many patients can become organ transplant targets. Therefore, there is a need to develop a preservation method that does not damage an organ even if the organ is preserved for a long time.

An exemplary method conducted for preserving a biological material such as an organ for a long time is the preservation of the same at low temperature, but tissue damage that exceeds the protective effect occurs when the low-temperature storage time exceeds a certain time. This tissue damage is called "cold preservation damage", "cold storage damage", or "rewarm damage" (these are hereinafter simply referred to as "cold damage"), and because of this cold damage, the upper limit of the cold storage time for an organ to be used for transplantation is determined depending on the organ.

The present disclosure, in one aspect, provides: a method for suppressing cold damage to a biological material such as an organ even if the biological material is preserved at low temperature for a long time; a composition for preventing cold damage; a method and a solution for preserving a biological material at low temperature; and a composition for preventing cold damage to a biological material. Means to Solve the Problem

The present disclosure, in one or a plurality of embodiments, relates to a method for suppressing cold damage to a biological material or a method for preserving the same at low temperature, the method including inhibiting a sphingosine-1-phosphoric acid (S1P)-mediated signal transduction pathway.

The present disclosure, in one or a plurality of embodiments, relates to a composition for preventing cold damage to a biological material or a solution for preserving a biological material, the composition or the solution containing, as an active ingredient, a substance that inhibits a S1P-mediatad signal transduction pathway (S1P signal transduction system inhibitor).

### Brief Description of Drawings

[FIG. 1] FIG. 1 is a graph illustrating exemplary results of assessment of cold damage suppression effects when a cell line HepG2 derived from a human liver cancer was cultured at low temperature under the presence of a compound 1 or 2 in Example 1.
[FIG. 2] FIG. 2 is a graph illustrating exemplary results of assessment of cold damage suppression effects when a cell line HepG2 derived from a human liver cancer, a human umbilical vascular endothelial cell line HUEhT-2, or a Flp-In™ CHO cell line was cultured under the presence of a compound 1 at low temperature in Example 2.
[FIG. 3] FIG. 3 illustrates exemplary results of assessment of cold damage suppression effects determined at varied timings of bringing cells preserved at low temperature into contact with the compound 1 in Example 3.
[FIG. 4] FIG. 4 is a graph illustrating exemplary viable and fibrotic areas of myocardium in the hearts of transplanted rats after the myocardium was preserved at low temperature (4 °C, 24 hours) in Example 4.
[FIG. 5] FIG. 5 is a graph illustrating exemplary results of assessment of cold damage suppression effects when a cell line HepG2 derived from a human liver cancer was cultured under the presence of one of compounds 1 and 3 to 7 at low temperature in Example 5.

### Mode for Carrying out the Invention

The present disclosure, in one aspect, is based on new findings that an S1P signal transduction pathway inhibiting substance such as an S1P receptor (S1PR) inhibitor or a sphingosine kinase (SPHK) inhibitor can suppress cold damage that occurs when cells, organs, or the like are preserved at low temperature.

The present disclosure, in one aspect, is also based on finding that low-temperature preservation of a biological material such as an organ after or while the biological material is in contact with an S1 PR inhibitor or an SPHK inhibitor makes it possible to preserve the biological material at low temperature for a long time while suppressing cold damage that occurs in the low-temperature preservation.

"Inhibiting a S1 P-mediated signal transduction pathway in a biological material" in the present disclosure is, in one or a plurality of embodiments, inhibiting the function of S1 PR, inhibiting the interaction between S1P and S1PR, inhibiting the production of S1P, inhibiting the production of S1PR, inhibiting the function of S1P, or the like. Inhibiting a S1P-mediated signal transduction pathway in a biological material, in one or a plurality of embodiments, can be performed by bringing the biological material into contact with a substance that inhibits a S1P-mediated signal transduction pathway. The substance that inhibits a S1P-mediated signal transduction pathway is, in one or a plurality of embodiments, an S1PR inhibiting substance such as S1PR antagonist, an S1P production inhibiting substance, an SPHK inhibiting substance, an S1P-S1PR interaction inhibiting substance, an S1P function inhibiting substance, or the like.

The "cold damage" in the present disclosure refers to damage occurring to a cell when a biological material such as a cell or an organ is exposed to a low temperature. Cold damage, in one or a plurality of embodiments, causes deterioration of cell viability, cell death, or the like. The "low temperature" in the present disclosure refers to, for example, a low temperature not below 0°C, and it is a temperature in a range of 0 to 4°C in one or a plurality of embodiments. In the present disclosure, the "prevention of cold damage", in one or a plurality of embodiments, encompasses suppressing cold damage, preventing cold damage, reducing cold damage, and the like. "Suppressing cold damage" in the present disclosure is, in one or a plurality of embodiments, suppressing deterioration of cell viability, suppressing cell death, or the like. The present disclosure, in one or a plurality of embodiments, can be applied to a biological material to which cold damage occurs in the case of low-temperature preservation, a biological material to which low-temperature preservation is not suitable, or a biological material that can be preserved at low temperature but is difficult to preserve at low temperature for a long time. The "long time" in the present disclosure varies depending on the type of the preserved biological material and the like; in one or a plurality of embodiments, it refers to a period longer than a period for which the biological material can be preserved by a conventional low-temperature preservation method, and particularly in one or a plurality of not particularly limiting embodiments, it refers to a period 1.5 times or more, twice or more, or three times or more the period for which the biological material can be preserved by a conventional low-temperature preservation method.

In one or a plurality of embodiments, examples of the biological material include animals such as a human or mammals other than a human, and biological materials derived from the animals (biological materials removed from the animals or biological materials of the animal origin). In one or a plurality of embodiments, examples of the biological material include organs, tissues, apparatuses, organisms, cells, body fluids, blood preparations, and cell sheets. In one or a plurality of embodiments, the apparatus and the organ is the heart, blood, skin, septum, cornea, liver, blood vessel, kidney, brain, eyeball, spleen, ovary, lung, intestine, nerve, placenta, umbilical cord, retina, or the like. Further, the organism is, for example, an animal such as a human or a mammal other than a human. In one or a plurality of embodiments, examples of the body fluid include blood and semen.

### [Composition for Preventing Cold Damage]

The present disclosure, in one or a plurality of embodiments, relates to a composition for preventing cold damage containing, as an active ingredient, a substance that inhibits an S1 P-mediated signal transduction pathway (cold damage prevention composition of the present disclosure). With the cold damage prevention composition of the present disclosure, in one or a plurality of embodiments, cold damage that would occur to a biological material when the material is preserved at low temperature can be suppressed. The cold damage prevention composition of the present disclosure, in one or a plurality of embodiments, can be used in a preservation solution or a perfusate for a biological material such as an organ used in transplantation. The cold damage prevention in the present disclosure, in one or a plurality of embodiments, encompasses suppressing cold damage, preventing cold damage, and the like.

The present disclosure makes it possible to suppress the deterioration of the condition of a biological material caused by cold damage in low-temperature preservation, thereby allowing for long-time low-temperature preservation; thus, the present disclosure makes it possible to keep the freshness of a biological material for a longer time.

### [Active Ingredient]

The cold damage prevention composition of the present disclosure contains, as an active ingredient, an S1P signal transduction pathway inhibiting substance. The S1P signal transduction pathway inhibiting substance is as described above, and in one or a plurality of non-limiting embodiments, examples of the same include an S1PR inhibiting compound (S1PR inhibitor) and an SPHK inhibiting compound (SPHK inhibitor).

The S1PR inhibiting compound is, in one or a plurality of embodiments, a compound that can inhibit the activity of S1PR in at least one of a known *in vitro* or *in vivo* S1PR inhibition assay system, in such a manner that the activity of S1PR when the compound is added can be inhibited to, for example, 60 % or less, 50 % or less, 40 % or less, 30 % or less, 20 % or less, or 10 % or less, as compared with the activity thereof in the control in which the foregoing compound is not added. In the foregoing assay system, the amount of the material added is 0.01 to 10 µM, in one or a plurality of embodiments. In one or a plurality of embodiments, the S1PR inhibiting compound may also have an inhibition activity against at least one of five types of receptors of S1PR (S1PR1, S1PR2, S1PR3, S1PR4, and S1PR5), or may have an inhibition activity against all of these five types.

The SPHK inhibiting compound is, in one or a plurality of embodiments, a compound that can inhibit the activity of SPHK in at least one of a known *in vitro* or *in vivo* SPHK inhibition assay system, in such a manner that the activity of SPHK when the compound is added can be inhibited to, for example, 60 % or less, 50 % or less, 40 % or less, 30 % or less, 20 % or less, or 10 % or less, as compared with the activity thereof in control in which the foregoing compound is not added. In the foregoing assay system, the amount of the material added is 0.01 to 10 µM, in one or a plurality of embodiments.

In one or a plurality of embodiments, the S1PR inhibiting compound used as an active ingredient in the cold damage prevention composition of the present disclosure is the compound expressed by Formula (I) or (I') shown below. Therefore, the cold damage prevention composition of the present disclosure, in another aspect, contains, as an active ingredient, the compound expressed by Formula (I) or (I') shown below, or a pharmaceutically acceptable salt of the compound. 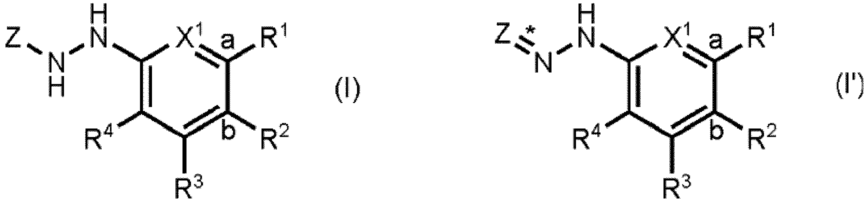 where
X¹ represents N or CR⁵,
R¹ and R² independently represent a hydrogen atom; a halogen atom, a C₁₋₈ alkyl group, a C₁₋₈ alkyl group substituted with a halogen atom, a C₁₋₈ alkoxy group, a C₂₋₈ alkynyl group, a C₁₋₈ alkylsulfonyl group, a C₁₋₈ alkyl amino sulfonyl group, a nitro group, or a cyano group, or alternatively, form an aromatic ring which may be substituted, or an aromatic heterocycle which may be substituted, together with atoms denoted by "a" and "b",
R³, R⁴, and R⁵ independently represent a hydrogen atom, a halogen atom, a C₁₋₈ alkyl group, a C₁₋₈ alkyl group substituted with a halogen atom, a C₁₋₈ alkoxy group, a C₂₋₈alkynyl group, a C₁₋₈alkylsulfonyl group, a C₁₋₈alkyl amino sulfonyl group, a nitro group, or a cyano group,
Z represents: 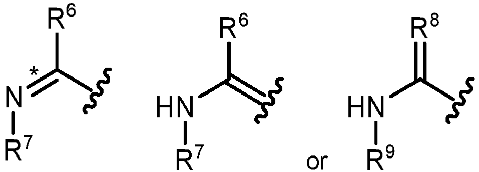
   where R⁶ represents -C(=O)R¹⁰ or -CH₂R¹¹,
      where R¹⁰ and R¹¹ each represent a C₂₋₈ alkyl group which may be substituted, a phenyl group which may be substituted, a C₂₋₈ alkoxy group which may be substituted, a heterocyclic aromatic group which may be substituted, or -NR¹²R¹³,
      where R¹² and R¹³ independently represent a hydrogen atom or a C₁₋₈ alkyl group which may be substituted, or alternatively, R¹² and R¹³ form a nitrogen-containing heterocyclic group which may be substituted, together with an adjacent nitrogen atom,
   R⁷ represents a phenyl group which may be substituted, a pyridinyl group which may be substituted, a quinoline group, or an isoquinoline group,
   R⁸ represents O or NR¹⁴,
      where R¹⁴ represents a hydrogen atom, a C₂₋₈ alkyl group which may be substituted, a phenyl group which may be substituted, a C₂₋₈ alkoxy group which may be substituted, or a heterocyclic aromatic group which may be substituted,
   R⁹ represents a phenyl group which may be substituted, a pyridinyl group which may be substituted, a quinoline group, or an isoquinoline group, and
   a bond to which wavy lines are attached represents a bonding part where the group is bonded with the compound expressed by Formula (I) or (I').

In Formula (I') and Z, the carbon-nitrogen double bond denoted by * represents a double bond having a structural isomer such as a stereoisomer or a geometrical isomer. With this double bond, the compounds expressed by Formulae (I) and (I') or a pharmaceutically acceptable salt of the same can include (E) alone, (Z) alone, (E) and (Z) in a mixed state, or a state where the stereoscopic structure is unspecified, as a geometrical isomerism formed with the carbon-nitrogen double bond.

In one or a plurality of embodiments, the halogen atom in R¹, R², R³, R⁴ and R⁵ is a fluorine atom, a chlorine atom, a bromine atom, or an iodine atom; preferably it is a fluorine atom, a chlorine atom, or a bromine atom; more preferably it is a chlorine atom.

In one or a plurality of embodiments, the C₁₋₈ alkyl group in R¹, R², R³, R⁴ and R⁵ is a linear or branched alkyl group having 1 to 8 carbon atoms, or a cyclic alkyl group having 3 to 7 carbon atoms. In one or a plurality of embodiments, the C₁₋₈ alkyl group is a methyl group, an ethyl group, an n-propyl group, an isopropyl group, an n-butyl group, an isobutyl group, a sec-butyl group, a tert-butyl group, an n-pentyl group, a 2-propyl pentyl group, an n-hexyl group, a cyclopentyl group, a cyclohexyl group, or the like.

In one or a plurality of embodiments, examples of the C₁₋₈ alkyl group substituted with a halogen atom in R¹, R², R³, R⁴ and R⁵ is a fluoromethyl group, a difluoromethyl group, a trifluoromethyl group, a chloromethyl group, a dichloromethyl group, a trichloromethyl group, a 2-chloro-1,1-dimethylethyl group, or the like; it is preferably a trifluoromethyl group.

In one or a plurality of embodiments, the C₁₋₈ alkoxy group in R¹, R², R³, and R⁴ is a linear or branched alkoxy group having 1 to 8 carbon atoms, or a cyclic alkyloxy group having 3 to 6 carbon atoms. In one or a plurality of embodiments, the C₁₋₈ alkoxy group is a methoxy group, an ethoxy group, an n-propoxy group, an isopropoxy group, an n-butoxy group, an isobutoxy group, a sec-butoxy group, a tert-butoxy group, an n-pentyloxy group, a 2-propylpentyloxy group, an n-hexyloxy group, a cyclopentyloxy group, a cyclohexyloxy group, or the like; it is preferably a methoxy group.

In one or a plurality of embodiments, the C₂₋₈ alkynyl group in R¹, R², R³, R⁴ and R⁵ is a linear, branched, or cyclic hydrocarbon group having 2 to 6 carbon atoms and having an unsaturated triple bond. In one or a plurality of embodiments, the C₂₋₈ alkynyl group is an ethynyl group, a 1-propynyl group, a 2-propynyl group, a 1-butynyl group, a 2-butynyl group, a 3-butynyl group, a 3-methyl-1-butynyl group, a 4-methyl-1-pentynyl group, or the like; it is preferably an ethynyl group.

In one or a plurality of embodiments, the C₁₋₈ alkylsulfonyl group in R¹, R², R³, R⁴ and R⁵ is a methanesulfonyl group, an ethanesulfonyl group, or the like; it is preferably a methanesulfonyl group.

In one or a plurality of embodiments, the C₁₋₈ alkyl amino sulfonyl group in R¹, R², R³, R⁴ and R⁵ is a dimethyl amino sulfonyl group, a diethyl amino sulfonyl group, or the like.

R¹ and R², together with atoms denoted by "a" and "b", may form an aromatic ring which may be substituted, or an aromatic heterocycle which may be substituted. In one or a plurality of embodiments, the aromatic ring is a benzene group, a naphthalene group, or the like. In one or a plurality of embodiments, the heterocyclic aromatic group is a 5 to 7-membered aromatic ring including one or two atoms of oxygen, nitrogen, sulfur atoms, or the like, which is, for example, a furyl group, a thienyl group, a pyrrolyl group, a pyridyl group, a pyrimidyl group, or the like. In one or a plurality of embodiments, the substituent group of the aromatic ring and the aromatic heterocycle is a halogen atom, a C₁₋₈ alkyl group, a C₁₋₈ alkyl group substituted with a halogen atom, a C₁₋₈ alkoxy group, or the like.

X¹ represents N or CR⁵, and preferably N or CH.

R⁶ represents -C(=O)R¹⁰ or -CH₂R¹¹, and preferably -C(=O)R¹⁰. R¹⁰ and R¹¹ each represents a C₂₋₈ alkyl group which may be substituted, a phenyl group which may be substituted, a C₂₋₈ alkoxy group which may be substituted, a heterocyclic aromatic group which may be substituted, or -NR¹²R¹³.

In one or a plurality of embodiments, the C₂₋₈ alkyl group which may be substituted, in R¹⁰ and R¹¹, is a linear or branched alkyl group that has 2 to 8 carbon atoms and that is unsubstituted or has a substituent group, or it is a cyclic alkyl group that has 3 to 7 carbon atoms and that is unsubstituted or has a substituent group. In one or a plurality of embodiments, the C₂₋₈ alkyl group represents an ethyl group, an n-propyl group, an isopropyl group, a 1 -ethylpropyl group, a 2,2-dimethylpropyl group, an n-butyl group, an isobutyl group, a sec-butyl group, a tert-butyl group, an n-pentyl group, a 2-propyl pentyl group, an n-hexyl group, a cyclopentyl group, a cyclohexyl group, or the like; preferably it represents an n-butyl group, a tert-butyl group, a 1-ethylpropyl group, or a 2,2-dimethylpropyl group. In one or a plurality of embodiments, the substituent group is a halogen atom, a C₁₋₈ alkoxy group, or the like.

In one or a plurality of embodiments, each of the phenyl group which may be substituted and the heterocyclic aromatic group which may be substituted, in R¹⁰ and R¹¹, is a phenyl group that is unsubstituted or has 1 to 3 substituent groups at arbitrary positions, or a heterocyclic aromatic group that is unsubstituted or has 1 to 3 substituent groups at arbitrary positions. In one or a plurality of embodiments, the heterocyclic aromatic group represents a 5 to 7-membered aromatic ring including one or two atoms such as oxygen, nitrogen, or sulfur atoms, which is, for example, a furyl group, a thienyl group, a pyrrolyl group, a pyridyl group, or a pyrimidyl group; it preferably represents a furyl group, a thienyl group, or a pyridyl group. In one or a plurality of embodiments, the substituent group is a halogen atom, a C₁₋₈ alkoxy group, or the like.

In one or a plurality of embodiments, the C₂₋₈ alkoxy group which may be substituted, in R¹⁰ and R¹¹, is a linear or branched alkoxy group that has 2 to 8 carbon atoms and that is unsubstituted or has a substituent group, or it is a cyclic alkyloxy group that has 3 to 6 carbon atoms and that is unsubstituted or has a substituent group. In one or a plurality of embodiments, the C₂₋₈ alkoxy group represents an ethoxy group, an n-propoxy group, an isopropoxy group, an n-butoxy group, an isobutoxy group, a sec-butoxy group, a tert-butoxy group, an n-pentyloxy group, a 2-propylpentyloxy group, an n-hexyloxy group, a cyclopentyloxy group, a cyclohexyloxy group, or the like; preferably it represents an ethoxy group. In one or a plurality of embodiments, the substituent group is a halogen atom, a C₁₋₈ alkoxy group, or the like.

In R¹² and R¹³, the C₁₋₈ alkyl group in the C₁₋₈ alkyl group which may be substituted is the same as the C₁₋₈ alkyl group indicated as those in R¹, R², R³, R⁴, and R⁵. In one or a plurality of embodiments, R¹² and R¹³ each are preferably a hydrogen atom or a tert-butyl group.

In a case where R¹² and R¹³ form a nitrogen-containing heterocyclic group which may be substituted, together with an adjacent nitrogen atom, the nitrogen-containing heterocycle in one or a plurality of embodiments is a 5 to 7-membered saturated heterocycle or a 5 to 7-membered unsaturated heterocycle. In one or a plurality of embodiments, the nitrogen-containing heterocycle is a pyrrole ring, a pyrrolidine ring, a pyrazole ring, a triazole ring, a piperazine ring, a piperidine ring, or the like; preferably it is a piperidine ring. In one or a plurality of embodiments, the substituent group is a halogen atom, a C₁₋₈ alkyl group, a phenyl group, a C₁₋₈ alkoxy group, or the like.

R⁷ represents a phenyl group which may be substituted, a pyridinyl group which may be substituted, a quinoline group, or an isoquinoline group. In one or a plurality of embodiments, the substituent group is a hydrogen atom, a halogen atom, a C₁₋₈ alkyl group, a C₁₋₈ alkoxy group, a C₂₋₈ alkynyl group, a C₁₋₈ alkylsulfonyl group, a cyano group, or the like. In one or a plurality of embodiments, R⁷ preferably represents a halogen atom, a trifluoromethyl group, a cyano group, a phenyl group substituted with a methoxy group or an ethynyl group, a phenyl group, a pyridinyl group, a pyridinyl group substituted with a halogen atom, a quinoline group, or an isoquinoline group; more preferably it is a phenyl group substituted with a halogen atom.

R⁸ represents O or NR¹⁴, where R¹⁴ represents a hydrogen atom, a C₂₋₈ alkyl group which may be substituted, a phenyl group which may be substituted, a C₂₋₈ alkoxy group which may be substituted, or a heterocyclic aromatic group which may be substituted; R⁸ preferably represents O.

R⁹ represents a phenyl group which may be substituted, a pyridinyl group which may be substituted, a quinoline group, or an isoquinoline group. In one or a plurality of embodiments, the substituent group is a hydrogen atom, a halogen atom, a C₁₋₈ alkyl group, a C₁₋₈ alkoxy group, a C₂₋₈ alkynyl group, a C₁₋₈ alkylsulfonyl group, a cyano group, or the like. In one or a plurality of embodiments, R⁹ preferably represents a pyridinyl group, or a pyridinyl group substituted with a halogen atom.

In the present disclosure, "which may be substituted" means not being substituted, or being substituted with a substituent group. The number of the substituent groups is not limited particularly; there may be one substituent group, or there may be two, three, or more substituent groups that are identical or different.

As described above, in one or a plurality of embodiments, the compound expressed by Formula (I) or (I') can include a carbon-nitrogen double bond having a stereoisomer. In one or a plurality of embodiments, the compound expressed by Formula (I) or (I'), therefore, is a compound expressed by any one of formulae below: 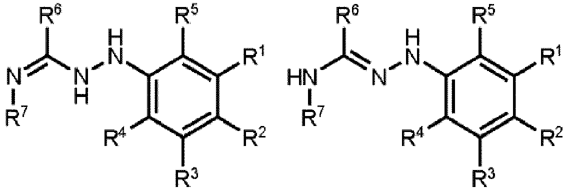 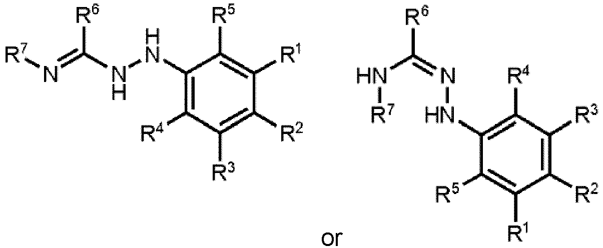 where R¹, R², R³, R⁴, R⁵, R⁶, and R⁷ are as described above.

In one or a plurality of embodiments, the compound expressed by Formula (I) or (I'), therefore, is a compound expressed by any one of formulae below: 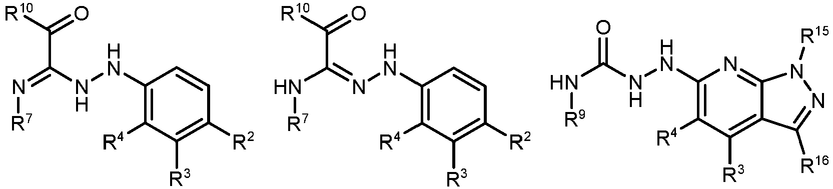 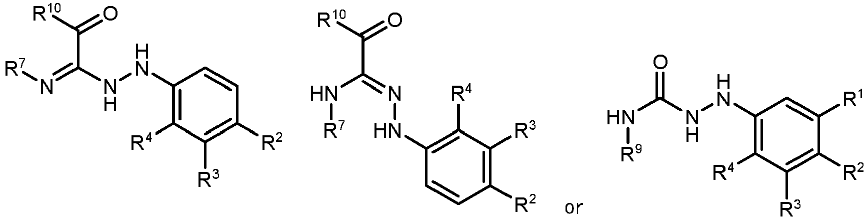 In the foregoing formulae,
R¹, R², R³, and R⁴ independently represent a hydrogen atom, a halogen atom, a methyl group, an ethyl group, an isopropyl group, a propyl group, a trifluoromethyl group, a cyano group, a methoxy group, an acetyl group, a methanesulfonyl group, or an ethynyl group; they preferably represent a hydrogen atom, a chlorine atom, a fluorine atom, a bromine atom, a trifluoromethyl group, an ethynyl group, or an isopropyl group.
R⁷ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group. Preferably R⁷ represents a halogen atom; a trifluoromethyl group; a cyano group; a phenyl group substituted with a methoxy group or an ethynyl group; or a phenyl group.
R⁹ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group.
R¹⁰ represents an n-butyl group, a tert-butyl group, an isobutyl group, a sec-butyl group, a 1-ethylpropyl group, a 2,2-dimethylpropyl group, a phenyl group which may be substituted, a furyl group which may be substituted, an ethoxy group, or -NR¹²R¹³. Here, R¹² and R¹³ independently represent a hydrogen atom or a tert-butyl group, or alternatively, R¹² and R¹³ together with an adjacent nitrogen atom form a piperidine ring. R¹² and R¹³ preferably independently represent an n-butyl group, a tert-butyl group, an isobutyl group, a sec-butyl group, a 1 -ethylpropyl group, or a 2,2-dimethylpropyl group.
R¹⁵ and R¹⁶ independently represent a hydrogen atom, a halogen atom, a methyl group, a trifluoromethyl group, a cyano group, a methoxy group, an acetyl group, a methanesulfonyl group, or an ethynyl group; they preferably represent a methyl group.

In one or a plurality of embodiments, the compound expressed by Formula (I) or (I') is a compound expressed by any one of formulae below:

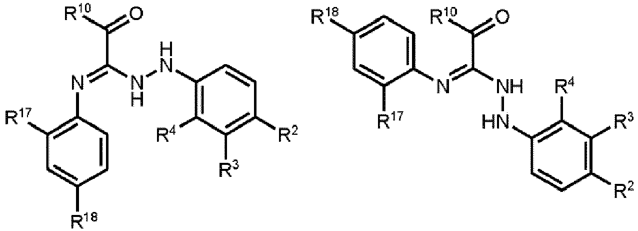

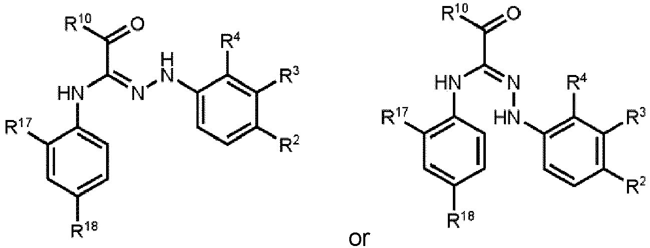

In the foregoing formulae,
R², R³, and R⁴ independently represent a hydrogen atom, a halogen atom, a methyl group, an ethyl group, an isopropyl group, a propyl group, a trifluoromethyl group, a cyano group, a methoxy group, an acetyl group, a methanesulfonyl group, or an ethynyl group; they preferably represent a hydrogen atom or a halogen atom; more preferably they represent a hydrogen atom or a chlorine atom. It is preferable that one of R², R³, and R⁴ is a halogen atom, and the other two are a hydrogen atom.
R¹⁰ represents a C₂₋₈ alkyl group which may be substituted, or a C₂₋₈ alkoxy group which may be substituted. Preferably they represent an n-butyl group, a tert-butyl group, an isobutyl group, a sec-butyl group, a 1-ethylpropyl group, a 2,2-dimethylpropyl group, an n-propyl group, an isopropyl group, a 1-ethylpropyl group, a 2,2-dimethylpropyl group, an n-butyl group, an isobutyl group, a sec-butyl group, a tert-butyl group, an n-pentyl group, a 2-propyl pentyl group, or an n-hexyl group. More preferably they represent an n-butyl group, a tert-butyl group, an isobutyl group, or a sec-butyl group.
R¹⁷ and R¹⁸ independently represent a hydrogen atom or a halogen atom; preferably they represent a halogen atom; more preferably they represent a chlorine atom or a fluorine atom.

In one or a plurality of embodiments, the compound expressed by Formula (I) or (I') is a compound expressed by any one of formulae below:

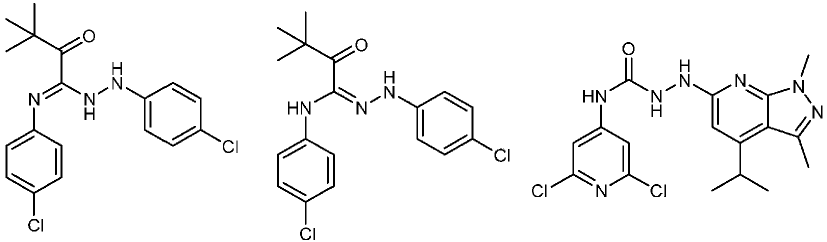

In one or a plurality of embodiments, the compound expressed by Formula (I) or (I') is a compound expressed by any one of formulae below:

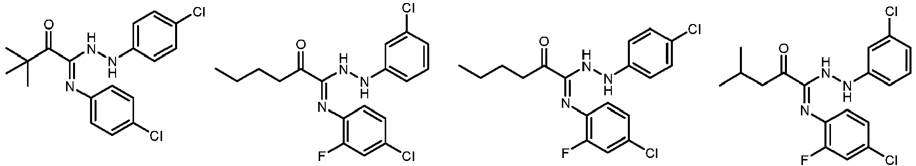

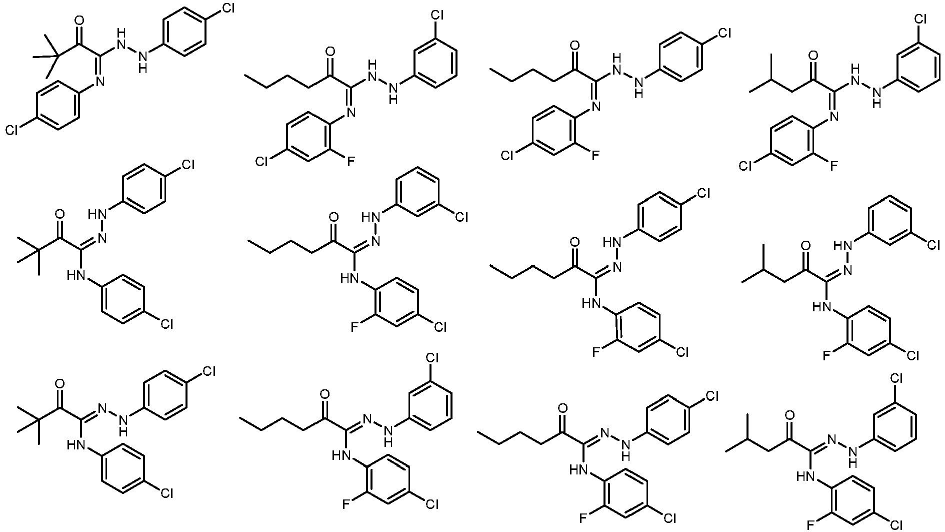

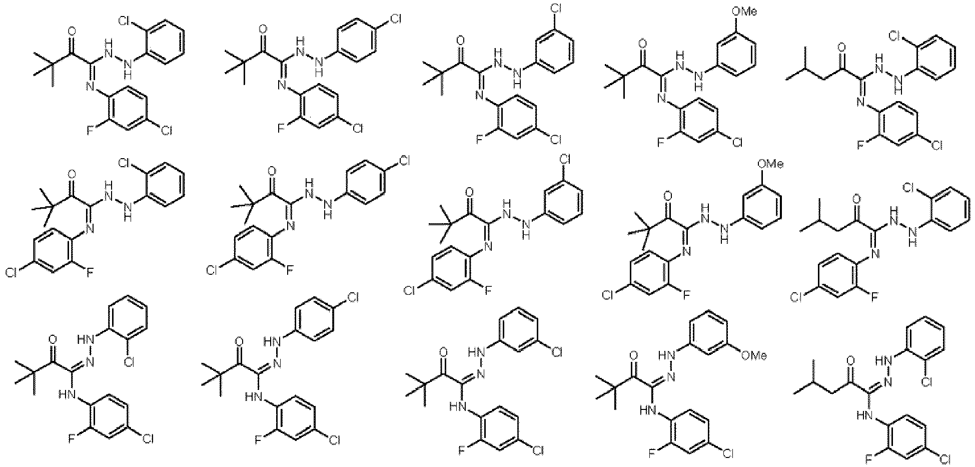

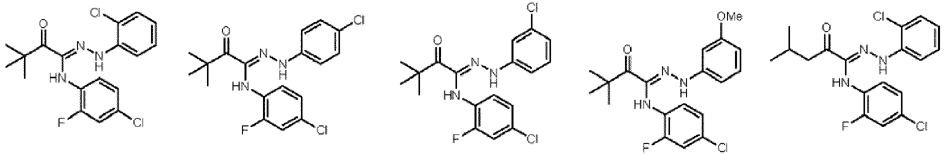

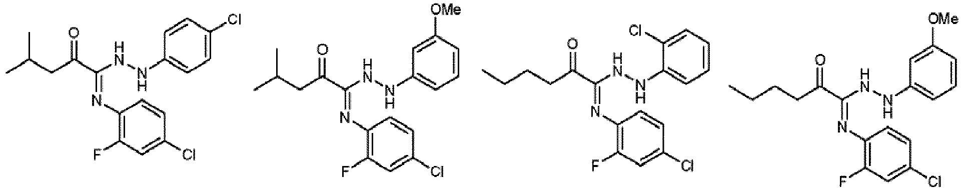

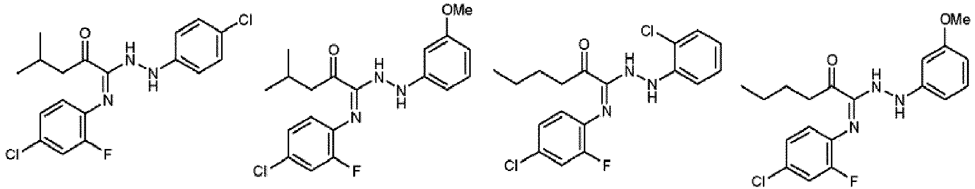

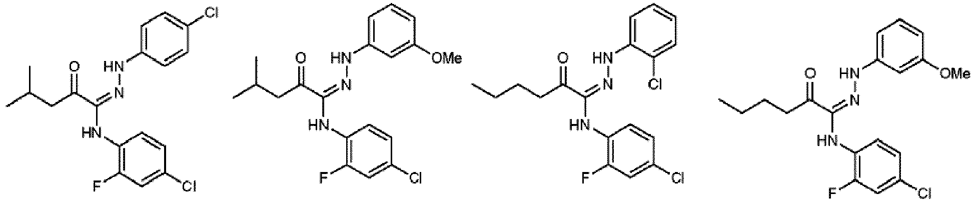

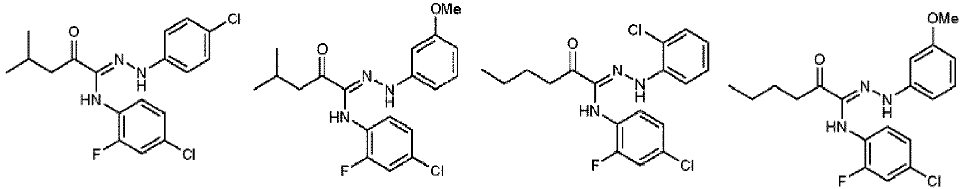

In one or a plurality of embodiments, the compound expressed by Formula (I) or (I') is a mixture of each isomer mixture or each isomer that is isolated, in a case where an asymmetric carbon atom is present and/or a stereoisomer is present. In one or a plurality of particularly non-limiting embodiments of the stereoisomer, the stereoisomer is a cis-trans isomer.

In the present disclosure, the "pharmaceutically acceptable salt" is a pharmacologically and/or pharmaceutically acceptable salt. It is, for example, an inorganic acid salt, an organic acid salt, an inorganic base salt, an organic base salt, an acidic amino acid salt, or a basic amino acid salt. In one or a plurality of embodiments, the inorganic acid salt is a hydrochloride, a hydrobromate, a sulfate, a nitrate, or a phosphate. In one or a plurality of embodiments, the organic acid salt is an acetate, a succinate, a fumarate, a maleate, a tartrate, a citrate, a lactate, a stearate, a benzoate, a methanesulfonate, or a p-toluenesulfonate. In one or a plurality of embodiments, the inorganic base salt is a salt of an alkali metal such as a sodium salt or a potassium salt, an alkali earth metal salt such as a magnesium salt or a calcium salt, an aluminum salt, or an ammonium salt. In one or a plurality of embodiments, the organic base salt is a diethylamine salt, a diethanolamine salt, a meglumine salt, or an N,N'-dibenzylethylenediamine salt. In one or a plurality of embodiments, the acidic amino acid salt is an aspartate, or a glutamate. In one or a plurality of embodiments, the basic amino acid salt is an arginine salt, a lysine salt, or an ornithine salt.

In the present disclosure, the "salt of a compound" may encompass a hydrate that can be formed when a compound, left in the atmosphere, absorbs moisture. Further, in the present disclosure, the "salt of a compound" may also encompass a solvate that can be formed when a compound absorbs a solvent of another kind.

In one or a plurality of embodiments, the cold damage prevention composition of the present disclosure contains the above-described inhibiting compound as an active ingredient, and may further contain a pharmaceutically acceptable carrier, preservative, diluent, excipient, or another pharmaceutically acceptable component.

In one or a plurality of embodiments, the cold damage prevention composition of the present disclosure may be liquid or solid. In one or a plurality of embodiments, in a case where the cold damage prevention composition of the present disclosure is liquid or solid, the cold damage prevention composition may further contain, for example: a physiological saline solution; a cell culture solution; a buffer solution; an infusion; a preservation solution for organs or the like; or a perfusate. In one or a plurality of embodiments, the cell culture solution is DMEM, MEM, or the like. In one or a plurality of embodiments, the buffer solution is a phosphoric acid buffer solution, a citric acid buffer solution, a tris buffer solution, an HEPES buffer solution, or the like. In one or a plurality of embodiments, the infusion is an electrolyte infusion, a nutrient infusion, a sugar infusion, an amino acid infusion, a glucose infusion, a Ringer's solution, an acetated Ringer's solution, a lactated Ringer's solution, or the like. In one or a plurality of embodiments, the preservation solution or the perfusate is a University of Wisconsin solution (UW solution), a Bretschneider solution, a Bretschneider's HTK solution, a Euro-Collins solution, a St. Thomas' Hospital solution, an ET-Kyoto solution, or the like.

The cold damage prevention composition of the present disclosure, in one or a plurality of embodiments, can be used as a composition for preserving a biological material such as organs and blood preparations, as well as a preservation solution for the same.

The present disclosure, in another aspect, therefore relates to a composition for preserving a biological material, a preservation solution, or a perfusate each of which contains an S1P signal transduction pathway inhibiting compound as an active ingredient. The composition for preservation, the preservation solution, or the perfusate of the present disclosure, in one or a plurality of embodiments, can contain, as an active ingredient, the compound expressed by Formula (I) or (I'), or pharmaceutically acceptable salts of the compound.

The S1P signal transduction pathway inhibiting compound as an active ingredient in the preservation solution or the perfusate of the present disclosure, in one or a plurality of non-limiting embodiments, has a concentration at which cold damage to a biological material can be suppressed; the concentration is in a range of 0.001 to 150 µM, in a range of 0.01 to 100 µM, or in a range of 0.1 to 100 µM.

The compound expressed by Formula (I) or (I'), or the pharmaceutically acceptable salt of the compound can suppress cold damage. Therefore, the present disclosure, in another aspect, relates to the cold damage prevention composition that contains, as an active ingredient, the compound expressed by Formula (I) or (I'), or a pharmaceutically acceptable salt of the compound. The content of the active ingredient, the form of the cold damage prevention composition, other components, and the like, are as described above.

[Method for Suppressing Cold Damage to Biological Material]

The present disclosure, in one or a plurality of embodiments, relates to a method for suppressing cold damage to a biological material (the cold damage suppression method of the present disclosure), the method including the step of inhibiting an S1 P-medeiated signal transduction pathway in the biological material. With the cold damage prevention method of the present disclosure, in one or a plurality of embodiments, it is possible to suppress the deterioration of cell viability, cell death, or the like that would occur to a biological material when the biological material is preserved at low temperature. The biological material is as described above.

The cold damage suppression method of the present disclosure, in one or a plurality of embodiments, includes the step of preserving the biological material at low temperature. The low-temperature preservation, in one or a plurality of embodiments, includes the preservation at low temperature not below 0°C, and can include the preservation at 0 to 4°C preferably.

The step of inhibiting a signal transduction pathway, in one or a plurality of embodiments, includes bringing the biological material into contact with a substance that inhibits the S1P signal transduction pathway. The substance that inhibits the S1P signal transduction pathway is as described above, and in one or a plurality of embodiments, examples of the same include an S1PR inhibiting compound and an SPHK inhibiting compound. In one or a plurality of embodiments, the S1 PR inhibiting compound is a compound expressed by Formula (I) or (I') shown above.

In one or a plurality of embodiments, the step of inhibiting a signal transduction pathway is performed before the start of the low-temperature preservation, simultaneously with the start of the low-temperature preservation, during the low-temperature preservation, or at any other timing.

The step of inhibiting a signal transduction pathway, in one or a plurality of embodiments, may be performed by immersing the biological material in a solution containing the S1P signal transduction pathway inhibiting compound, or by applying, adding, dropping, or atomizing the solution to the biological material. The cold damage suppression method of the present disclosure, in one or a plurality of particularly non-limiting embodiments, includes the step of immersing the biological material in a solution containing the compound expressed by Formula (I) or (I') shown above. In one or a plurality of embodiments, the solution may further contain a physiological saline solution, a cell culture solution, a buffer solution, an infusion, a preservation solution for organs or the like, or a perfusate, in addition to the S1P signal transduction pathway inhibiting compound or the compound expressed by Formula (I) or (I') as an active ingredient.

In a case where the biological material is an organ or an apparatus, the cold damage suppression method of the present disclosure, in one or a plurality of embodiments, may include at least one of perfusion, immersion, rinsing, and injection with the preservation solution or the perfusate containing the compound expressed by Formula (I) or (I'), as well as a combination of any of these.

The compound expressed by Formula (I) or (I'), or the pharmaceutically acceptable salt of the compound can suppress cold damage. Therefore, the present disclosure, in another aspect, relates to a method for suppressing cold damage to a biological material, the method including the step of bringing the compound expressed by Formula (I) or (I'), a pharmaceutically acceptable salt of the compound, or a composition containing any of the same, into contact with the biological material. The contact between the biological material and the compound expressed by Formula (I) or (I') or the like, in one or a plurality of embodiments, may be achieved by immersing the biological material in a solution containing the compound expressed by Formula (I) or (I'), or by applying, adding, dropping, or atomizing the solution to the biological material.

[Method for Preserving Biological Material]

The present disclosure, in one or a plurality of embodiments, relates to a method for preserving a biological material (the preservation method of the present disclosure), the method including the step of preserving, at low temperature, the biological material having been brought in contact with at least one of S1P signal transduction pathway inhibiting compounds. With the preservation method of the present disclosure, in one or a plurality of embodiments, it is possible to preserve the biological material at low temperature, while suppressing cold damage. The preservation method of the present disclosure makes it possible to allow for long-time low-temperature preservation, thereby making it possible to keep the freshness of a biological material for a longer time. The preservation method of the present disclosure, in one or a plurality of embodiments, can be used for preservation or the like of the biological material such as an organ used in transplantation.

In the preservation method of the present disclosure, in one or a plurality of embodiments, the contact between the biological material and the S1P signal transduction pathway inhibiting compound may be achieved before starting storage (preservation) of the biological material at low-temperature, or simultaneously with the start of storage (preservation) of the biological material at low-temperature, with a view to suppressing cold damage sufficiently and enabling the low-temperature preservation for a longer time. The preservation method of the present disclosure, in one or a plurality of embodiments, may include the step of preserving the biological material at low temperature while bringing the biological material and the S1P signal transduction pathway inhibiting compound in contact with each other. The preservation temperature may be a low temperature not below 0°C, and is a temperature in a range of 0 to 4°C in one or a plurality of embodiments. The contact between the biological material and the S1P signal transduction pathway inhibiting compound, in one or a plurality of embodiments, may be achieved by immersing the biological material in a solution containing the S1P signal transduction pathway inhibiting compound, or by applying, adding, dropping, or atomizing the solution to the biological material. In a case where the biological material is an organ or an apparatus, the contact between the biological material and the S1P signal transduction pathway inhibiting compound, in one or a plurality of embodiments, may be achieved by at least one of perfusion, immersion, rinsing, and injection with the preservation solution or the perfusate containing the compound expressed by Formula (I) or (I'), as well as a combination of any of these.

The compound expressed by Formula (I) or (I'), or the pharmaceutically acceptable salt of the compound can suppress cold damage. Therefore, the present disclosure, in another aspect, relates to a method for preserving the biological material at low temperature, the biological material having been brought into contact with the compound expressed by Formula (I) or (I'), a pharmaceutically acceptable salt of the compound, or a composition containing any of the same. The contact between the biological material and the compound expressed by Formula (I) or (I') or the like, the temperature for preserving the biological material, and the like, are as described above.

In other words, the present disclosure may relate to one or a plurality of embodiments described below:
[A1] A cold damage suppression method for suppressing cold damage to a biological material, the method including the step of inhibiting an S1 P-medeiated signal transduction pathway in the biological material.
[A2] The cold damage suppression method according to Item [A1], wherein the step of inhibiting a signal transduction pathway includes bringing the biological material into contact with a substance that inhibits the S1P signal transduction pathway.
[A3] The cold damage suppression method according to Item [A2],
   wherein the substance that inhibits the S1P signal transduction pathway is at least one selected from the group consisting of an S1 PR inhibiting compound and an SPHK inhibiting compound.
[A4] The cold damage suppression method according to any one of Items [A1] to [A3], the method further including the step of preserving the biological material at low temperature,
   wherein the step of inhibiting a signal transduction pathway is carried out before the step of preserving the biological material at low temperature, or simultaneously with the start of the step of preserving the biological material at low temperature.
[A5] The cold damage suppression method according to Item [A3],
   wherein the S1PR inhibiting compound is a compound expressed by Formula (I) or (I') below, or a pharmaceutically acceptable salt of the compound: 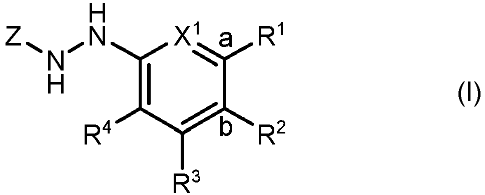 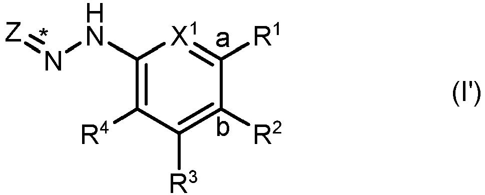
   where X¹ represents N or CR⁵,
   R¹ and R² independently represent a hydrogen atom; a halogen atom, a C₁₋₈ alkyl group, a C₁₋₈ alkyl group substituted with a halogen atom, a C₁₋₈ alkoxy group, a C₂₋₈ alkynyl group, a C₁₋₈ alkylsulfonyl group, or a cyano group, or alternatively, R¹ and R² form an aromatic ring which may be substituted, or an aromatic heterocycle which may be substituted, together with atoms denoted by "a" and "b",
   R³, R⁴, and R⁵ independently represent a hydrogen atom, a halogen atom, a C₁₋₈ alkyl group, a C₁₋₈ alkyl group substituted with a halogen atom, a C₁₋₈ alkoxy group, a C₂₋₈ alkynyl group, a C₁₋₈ alkylsulfonyl group, or a cyano group,
   Z represents: 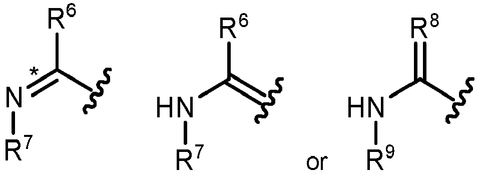
      where R⁶ represents -C(=O)R¹⁰ or -CH₂R¹¹, where R¹⁰ and R¹¹ each represent a C₂₋₈ alkyl group which may be substituted, a phenyl group which may be substituted, a C₂₋₈ alkoxy group which may be substituted, a heterocyclic aromatic group which may be substituted, or -NR¹²R¹³,
         where R¹² and R¹³ independently represent a hydrogen atom or a C₁₋₈ alkyl group which may be substituted, or alternatively, R¹² and R¹³ together with an adjacent nitrogen atom form a nitrogen-containing heterocyclic group which may be substituted,
      R⁷ represents a phenyl group which may be substituted, a pyridinyl group which may be substituted, a quinoline group, or an isoquinoline group,
      R⁸ represents O or NR¹⁴,
         where R¹⁴ represents a hydrogen atom, a C₂₋₈ alkyl group which may be substituted, a phenyl group which may be substituted, a C₂₋₈ alkoxy group which may be substituted, or a heterocyclic aromatic group which may be substituted, and
      R⁹ represents a phenyl group which may be substituted, a pyridinyl group which may be substituted, a quinoline group, or an isoquinoline group.
[A6] The cold damage suppression method according to Item [A5],
   wherein the compound expressed by Formula (I) or (I') is a compound expressed by: 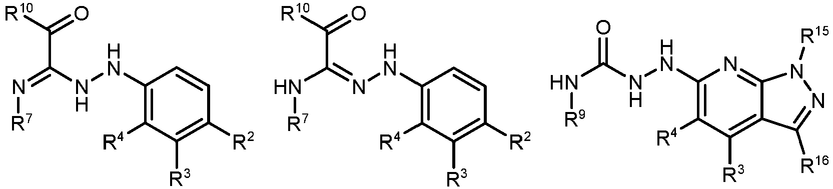 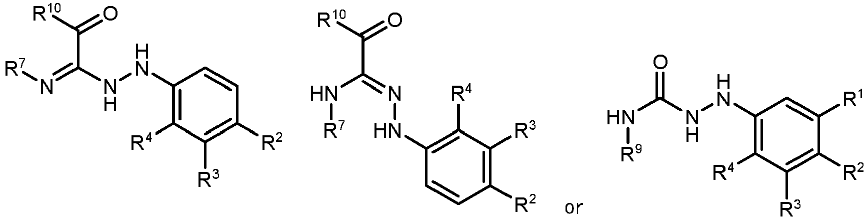
   where R¹, R², R³, and R⁴ independently represent a hydrogen atom, a halogen atom, a methyl group, an ethyl group, an isopropyl group, a propyl group, a trifluoromethyl group, a cyano group, a methoxy group, an acetyl group, a methanesulfonyl group, or an ethynyl group,
   R⁷ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group,
   R⁹ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group,
   R¹⁰ represents an n-butyl group, a tert-butyl group, a 1-ethylpropyl group, a 2,2-dimethylpropyl group, a phenyl group which may be substituted, a furyl group which may be substituted, an ethoxy group, or -NR¹²R¹³,
      where R¹² and R¹³ independently represent a hydrogen atom or a tert-butyl group, or alternatively, R¹² and R¹³ together with an adjacent nitrogen atom form a piperidine ring, and
   R¹⁵ and R¹⁶ independently represent a hydrogen atom, a halogen atom, a methyl group, a trifluoromethyl group, a cyano group, a methoxy group, an acetyl group, a methanesulfonyl group, or an ethynyl group.
[A7] The cold damage suppression method according to Item [A5] or [A6],
   wherein the compound expressed by Formula (I) or (I') is a compound expressed by: 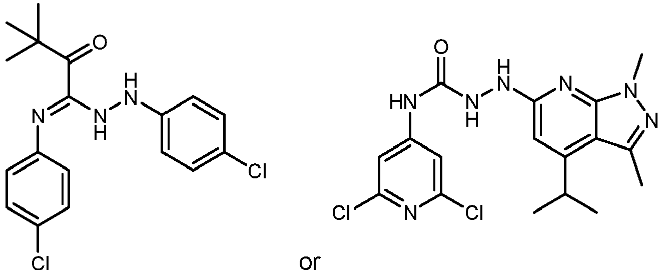
[A8] The cold damage suppression method according to any one of Items [A1] to [A7], wherein the biological material is at least one selected from the group consisting of organs, tissues, cells, body fluids, blood preparations, and cell sheets.
[A9] A cold damage prevention composition containing, as an active ingredient, a substance that inhibits an S1P-mediated signal transduction pathway.
[A10] The cold damage prevention composition according to Item [A9],
   wherein the substance is a compound expressed by Formula (I) or (I') below, or a pharmaceutically acceptable salt of the compound: 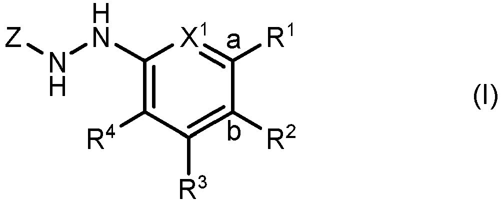 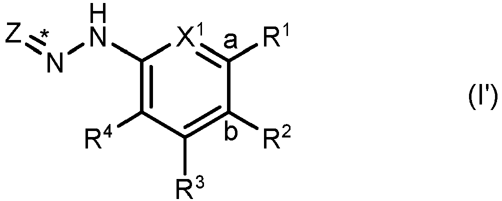
   where X¹ represents N or CR⁵,
   R¹ and R² independently represent a hydrogen atom; a halogen atom, a C₁₋₈ alkyl group, a C₁₋₈ alkyl group substituted with a halogen atom, a C₁₋₈ alkoxy group, a C₂₋₈ alkynyl group, a C₁₋₈ alkylsulfonyl group, or a cyano group, or alternatively, R¹ and R² form an aromatic ring which may be substituted, or an aromatic heterocycle which may be substituted, together with atoms denoted by "a" and "b",
   R³, R⁴, and R⁵ independently represent a hydrogen atom, a halogen atom, a C₁₋₈ alkyl group, a C₁₋₈ alkyl group substituted with a halogen atom, a C₁₋₈ alkoxy group, a C₂₋₈ alkynyl group, a C₁₋₈ alkylsulfonyl group, or a cyano group, and
   Z represents: 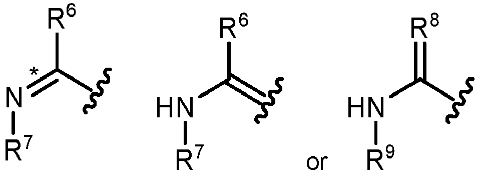
      where R⁶ represents -C(=O)R¹⁰ or -CH₂R¹¹, where R¹⁰ and R¹¹ each represent a C₂₋₈ alkyl group which may be substituted, a phenyl group which may be substituted, a C₂₋₈ alkoxy group which may be substituted, a heterocyclic aromatic group which may be substituted, or -NR¹²R¹³,
         where R¹² and R¹³ independently represent a hydrogen atom or a C₁₋₈ alkyl group which may be substituted, or alternatively, R¹² and R¹³ together with an adjacent nitrogen atom form a nitrogen-containing heterocyclic group which may be substituted,
      R⁷ represents a phenyl group which may be substituted, a pyridinyl group which may be substituted, a quinoline group, or an isoquinoline group,
      R⁸ represents O or NR¹⁴,
         where R¹⁴ represents a hydrogen atom, a C₂₋₈ alkyl group which may be substituted, a phenyl group which may be substituted, a C₂₋₈ alkoxy group which may be substituted, or a heterocyclic aromatic group which may be substituted, and
      R⁹ represents a phenyl group which may be substituted, a pyridinyl group which may be substituted, a quinoline group, or an isoquinoline group.
[A11] The cold damage prevention composition according to Item [A10],
   wherein the compound expressed by Formula (I) or (I') below: 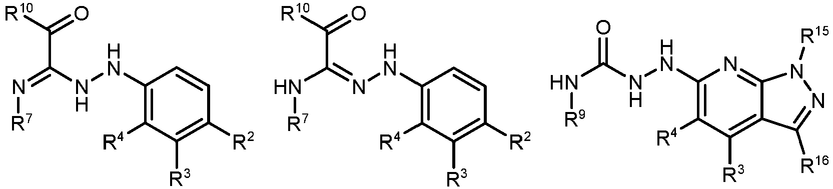 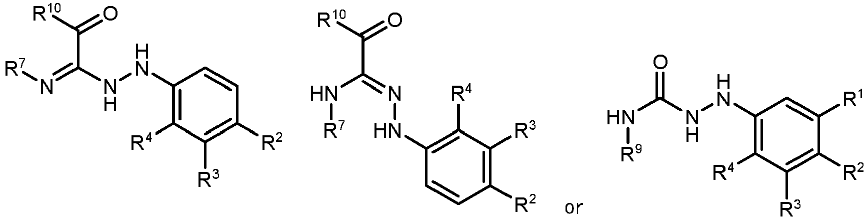
   where R¹, R², R³, and R⁴ independently represent a hydrogen atom, a halogen atom, a methyl group, an ethyl group, an isopropyl group, a propyl group, a trifluoromethyl group, a cyano group, a methoxy group, an acetyl group, a methanesulfonyl group, or an ethynyl group,
   R⁷ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group,
   R⁹ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group,
   R¹⁰ represents an n-butyl group, a tert-butyl group, a 1-ethylpropyl group, a 2,2-dimethylpropyl group, a phenyl group which may be substituted, a furyl group which may be substituted, an ethoxy group, or -NR¹²R¹³,
      where R¹² and R¹³ independently represent a hydrogen atom or a tert-butyl group, or alternatively, R¹² and R¹³ together with an adjacent nitrogen atom form a piperidine ring, and
   R¹⁵ and R¹⁶ independently represent a hydrogen atom, a halogen atom, a methyl group, a trifluoromethyl group, a cyano group, a methoxy group, an acetyl group, a methanesulfonyl group, or an ethynyl group.
[A12] A cold damage prevention composition containing, as an active ingredient, a compound expressed by: 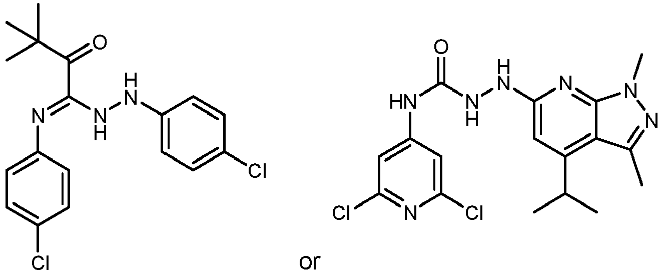
[A13] A method for preserving a biological material at low temperature, the method including the step of inhibiting an A1 P-mediated signal transduction pathway in the biological material.
[A14] A solution for preserving a biological material, the solution containing, as an active ingredient, a substance that inhibits an S1P-mediated signal transduction pathway.
[A15] The solution according to Item [A14],
   wherein the substance is the compound according to any one of Items [A9] to [A11].
[B1] A cold damage suppression method for suppressing cold damage to a biological material, the method comprising the step of bringing the biological material into contact with the compound expressed by Formula (I) or (I') according to any one of [A9] to [A11], a pharmaceutically acceptable salt of the compound, or a composition containing any of these.
[B2] The cold damage suppression method according to Item [B1], the method further comprising the step of preserving the biological material at low temperature,
   wherein the contact is achieved before the step of preserving the biological material at low temperature, or simultaneously with the start of the step of preserving the biological material at low temperature.
[B3] The cold damage suppression method according to Item [B1] or [B2],
   wherein the biological material is at least one selected from the group consisting of organs, tissues, cells, body fluids, blood preparations, and cell sheets.
[B4] A cold damage prevention composition containing, as an active ingredient, the compound expressed by Formula (I) or (I') according to any one of [A9] to [A11], or a pharmaceutically acceptable salt of the compound.
[B5] A method for preserving a biological material at low temperature, the method comprising the step of bringing the biological material into contact with the compound expressed by Formula (I) or (I') according to any one of [A9] to [A11], a pharmaceutically acceptable salt of the compound, or a composition containing any of these.
[B6] A solution for preserving a biological material, the solution containing, as an active ingredient, the compound expressed by Formula (I) or (I') according to any one of [A9] to [A11], or a pharmaceutically acceptable salt of the compound.

### [EXAMPLE]

Hereinafter, although the following description describes the present disclosure in more detail by way of examples, these are illustrative, and the present disclosure is not limited to these examples. Note that all of the references cited in the present disclosure are incorporated as a portion of the present disclosure.

[Production Example]

Compounds 1 and 3 to 7 were synthesized according to the following schemes.

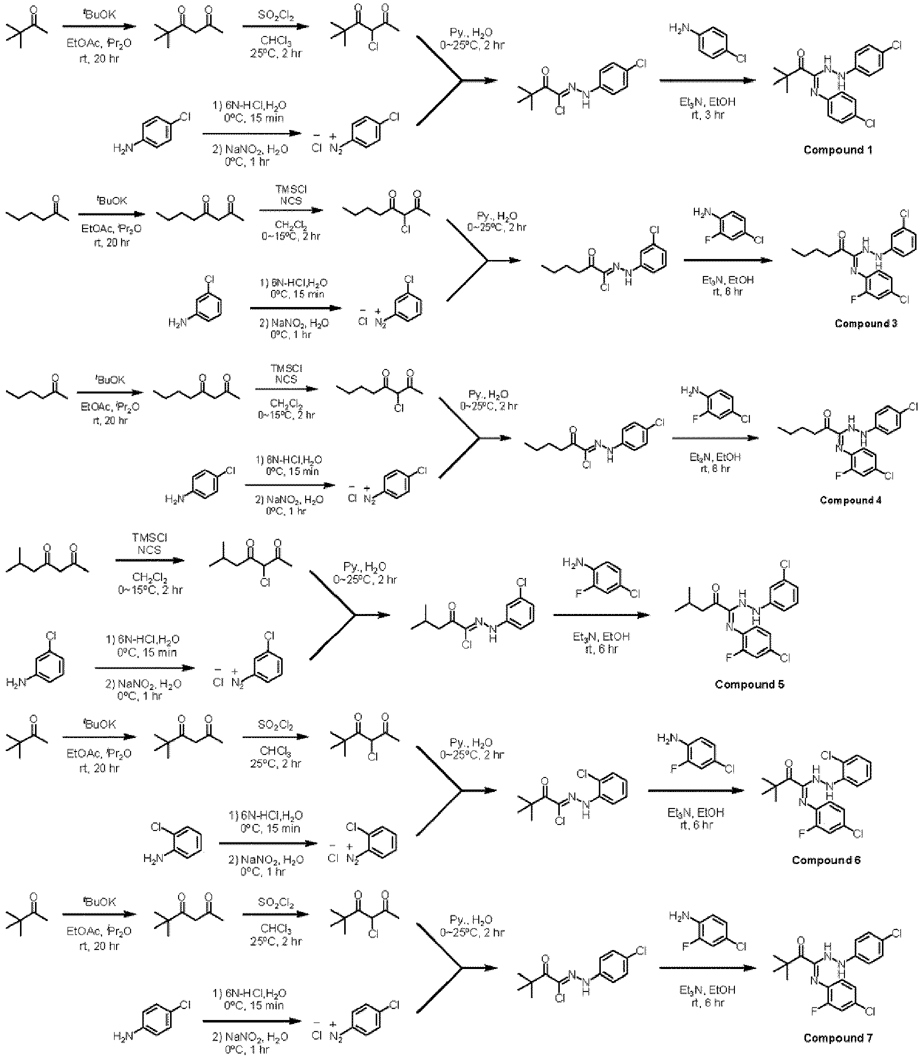

The compounds 1 and 3 to 7 thus synthesized have spectrum data (¹H-NMR and MS) as shown below.

| | Structural formula | ¹H-NMR(500MHz) | MS(ESI-TOF) |
|---|---|---|---|
| Compound 1 | 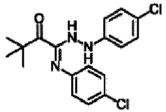 | (CDCl₃) 1.39(s, 1H), 6.53(d, *J*=8.8Hz, 2H), 7.1 9(d, *J*=8.8Hz, 2H), 7.26(d, *J*=8.8Hz, 2H), 7.33 (d, *J*=9.1Hz, 2H), 7.89(brs, 1H), 9.82(brs, 1H) | 386(M+Na) |
| Compounds | 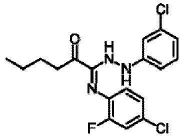 | (CDCl₃) 0.97(t, *J*=7.5Hz, 3H), 1.39-1.46(m, 2 H), 1.68-1.74(m. 2H), 3.04(t, *J*=7.5Hz, 2H), 6. 19(t, *J*=8.8Hz, 1H), 6.67(brs, 1H), 6.92-6.99 (m, 3H), 7.15-7.17(m, 2H), 7.21(t, *J*=8,1Hz, 1 H), 7.31(brs, 1H) | 404(M+Na) |
| Compound 4 | 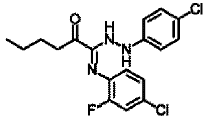 | (CDCl₃) 0.97(t, *J*=7.4Hz, 3H), 1.38-1.46(m, 2 H), 1.67-1.73(m, 2H), 3.03(t, *J*=7.5Hz, 2H), 6. 18(t, *J*=8.7Hz, 1H), 6.64(brs, 1H), 6,98(d, *J*= 8.6Hz, 1H), 7.06(d, *J*=8,6Hz, 2H), 7.16(dd, *J*= 10.3, 2.3Hz, 1H), 7.27(d, *J*=8.5Hz, 2H), 7.33 (brs, 1H) | 404(M+Na) |
| Compound 5 | 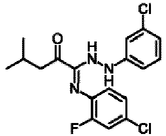 | (CDCl₃) 1.02(d, *J*=6.5Hz, 6H), 2.24-2.32(m, 1 H), 2.91(d, *J*=7.0Hz, 2H), 6.18(t, *J*=8.7Hz, 1 H), 6.69(brs, 1H), 6.93-6.99(m, 3H), 7.15-7.18 (m, 2H), 7.22(t, *J*=8, 1Hz, 1H), 7.32(brs, 1H) | 404(M+Na) |
| Compound 6 | 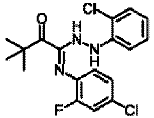 | (DMSO-d6) 1.26(s, 9H), 5.47(d, *J*=1.8Hz, 1H), 6.70-6.76(m, 2H), 7.39-7.42(m, 1H), 7.54-7.64 (m, 3H), 8.61(brs, 1H) | 383(M+2H) |
| Compound 7 | 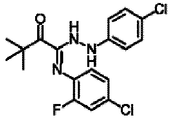 | (DMSO-d6) 1.29(s, 9H), 6.17(d, *J*=1.9Hz, 1H), 6.73-6.81(m, 2H), 7.42(d, *J*=8.7Hz, 2H), 7.53 (d, *J*=8.SHz, 2H), 8.64(brs, 1H) | 383(M+2H) |

In Examples 1 to 4 below, the compounds 1 and 2 were used. The compounds 1 and 2 are known as S1PR antagonists. It has been also reported that the compound 1 has an inhibiting activity to any of S1PR1, S1PR2, S1PR3, S1PR4, and S1PR5, and has selectivities to S1PR3, S1PR1, S1PR4, S1PR5, and S1PR2 in the descending order. The compound 2 can be synthesized by a known method.

### Compound 1

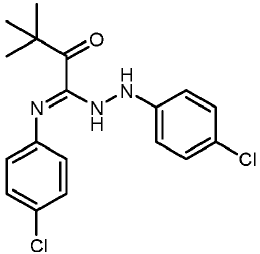

(N-(4-chlorophenyl)-N'-(4-chlorophenylamino)-3,3-dimethyl-2-oxobutanamidine

### Compound 2

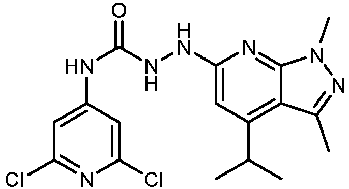

N-(2,6-dichloro-4-pyridinyl)-2-[1,3-dimethyl-4-(1-methylethyl)-1H-pyrazolo[3,4-b]pyridin-6-yl]-hydrazinecarboxamide

[Example 1: Assessment 1 of Cold Damage Suppression Effects]

Assessed were cold damage suppression effects when a cell line HepG2 derived from a human liver cancer was cultured under the presence of a compound 1 or 2 at low temperature by the following procedures.

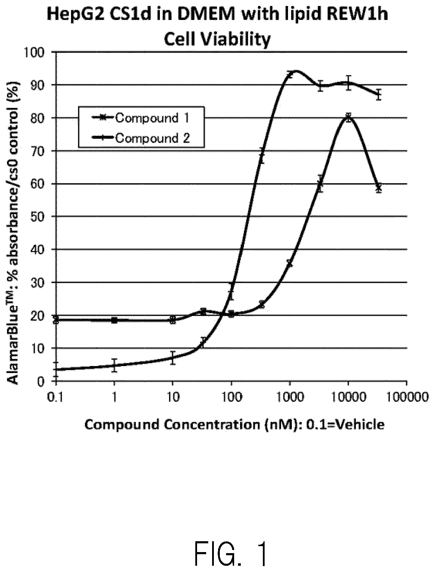

HepG2 cells were cultured at 37°C so as to be at a density of 80 to 90 %, and thereafter, the medium was replaced with a medium (DMEM) at 4°C to which the compound 1 or 2 had been added preliminarily. Then, the culturing at 4°C in a low temperature incubator was performed for 24 hours. After the culturing at low temperature for 24 hours, rewarming was carried out using a medium at 37°C, and the viability of cells one hour after the rewarming was determined by detecting enzyme activity using alamarBlue™ The compound concentration was varied from 0 M (Vehicle) to 50 µM, so that the concentration dependency of the compound was observed. Exemplary results thereof are shown in FIG. 1.

As illustrated in FIG. 1, the low-temperature preservation under the presence of the compound 1 or 2 provides the concentration-dependent amelioration of the deterioration of cell viability during the low-temperature preservation, thereby suppressing cold damage. Further, the compounds 1 and 2, which are S1 PR receptor inhibitors, did not exhibit toxicity when administered to the cells at a time other than the low-temperature preservation, for example, the compounds 1 and 2 did not exhibit toxicity when administered to the cells during a normal-temperature preservation.

An immortalized human umbilical vascular endothelial cell line HUEhT-21 was cultured in an SPHK inhibitor-added medium at low temperature (4°C) for five days. As a result, the deterioration of cell viability after rewarming to 37°C could be suppressed, as compared with a case of the low-temperature culturing in a control (a medium in which the SPHK inhibitor was not added).

[Example 2: Assessment 2 of Cold Damage Suppression Effects]

Assessed were cold damage suppression effects when a cell line HepG2 derived from a human liver cancer, a human umbilical vascular endothelial cell line HUEhT-2, or a Flp-In™ CHO cell line was cultured under the presence of the compound 1 at low temperature by the following procedures.

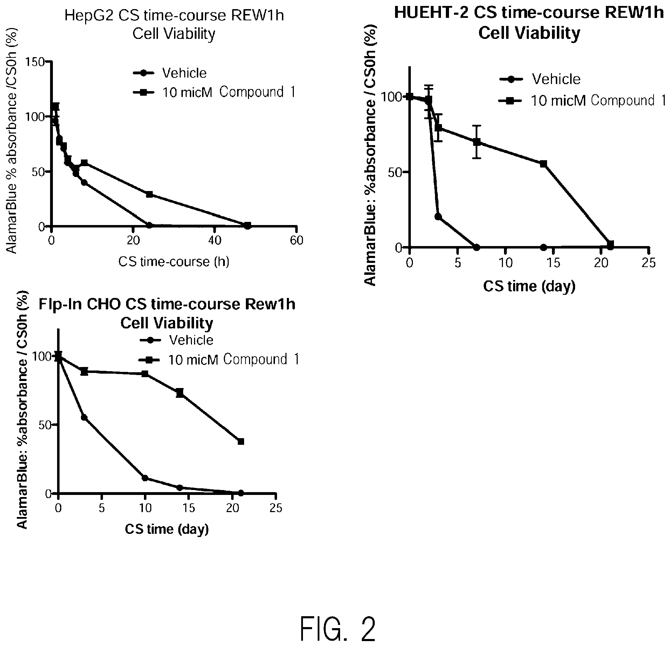

Cells were cultured at 37°C so as to be at a density of 80 to 90 %, and thereafter, the medium was replaced with Vehicle (a medium at 4°C) or the medium to which 10 µM of the compound 1 had been added preliminarily. Then, the culturing at 4°C in a low temperature incubator was performed. The cell viability one hour after the rewarming using a medium at 37°C was determined by detecting enzyme activity with use of alamarBlue™ The relationship between the cold storage time and the viability was observed. Exemplary results thereof are shown in FIG. 2.

As illustrated in FIG. 2, the low-temperature preservation under the presence of the compound 1 provided the amelioration of the deterioration of cell viability during the low-temperature preservation, regarding every cell line, thereby suppressing cold damage. Particularly regarding HUEhT-2 and Flp-In™ CHO, the viabilities thereof in the control (Vehicle) was lower than 20 % when the low-temperature preservation period exceeded three days or ten days; on the other hand, in the case of the low-temperature preservation under the presence of the compound 1, the viabilities thereof exceeded 50 % even when the low-temperature preservation period was 15 days. In other words, it was confirmed that the low-temperature preservation under the presence of the compound 1 made it possible to significantly extend the period of low-temperature preservation.

[Example 3: Assessment 3 of Cold Damage Suppression Effects]

Assessed were cold damage suppression effects determined at varied timings of bringing cells preserved at low temperature into contact with the compound 1 by the following procedures.

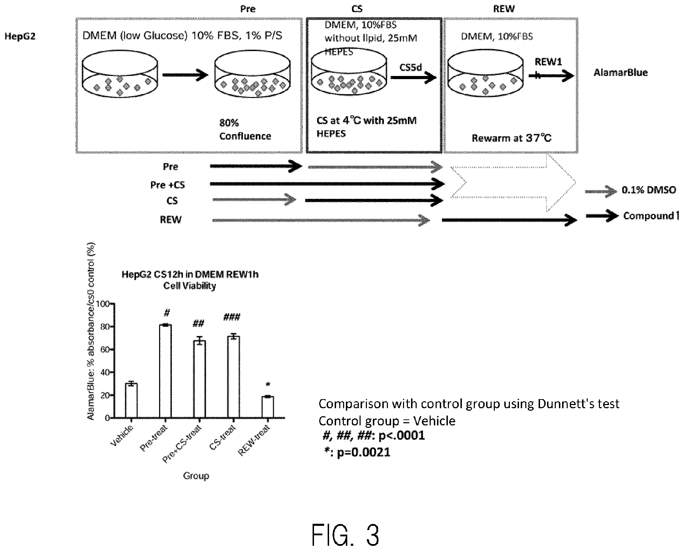

Cells were cultured at 37°C so as to be at a density of 80 to 90 %, and thereafter, the medium was replaced with a medium at 4°C. Then, the culturing at 4°C in a low temperature incubator was performed. For the Pre treat group, a medium in which 10 µM of the compound 1 was added was used from one hour before the cold storage till immediately before the cold storage; for the Pre+CS treat group, a medium in which 10 µM of the compound 1 was added was used from one hour before the cold storage during the cold storage, till immediately before the rewarming; for the CS treat group, a medium in which 10 µM of the compound 1 was added was used, the use being started simultaneously with the cold storage, till immediately before the rewarming; for the Rewarm (REW) treat group, a medium in which 10 µM of the compound 1 was added was used, the use being started simultaneously with the rewarming at 37°C. During a period other than the above-described periods, a medium having the composition illustrated in FIG. 3 (not containing the compound 1) was used for culturing. The cell viability one hour after the rewarming using a medium at 37°C was determined by detecting enzyme activity with use of alamarBlue™ Exemplary results thereof are shown in FIG. 3.

As illustrated in FIG. 3, cold damage (deterioration of viability) was not sufficiently suppressed in a case where the compound 1 was added to the medium at a timing when the temperature was turned to a normal temperature (37 °C) after the low-temperature preservation. On the other hand, by adding the compound 1 to the medium before the start of the low-temperature preservation or simultaneously with the start of the low-temperature preservation, cold damage (deterioration of viability) caused by the storage at low temperature could be suppressed.

[Example 4: Assessment 4 of Cold Damage Suppression Effects]

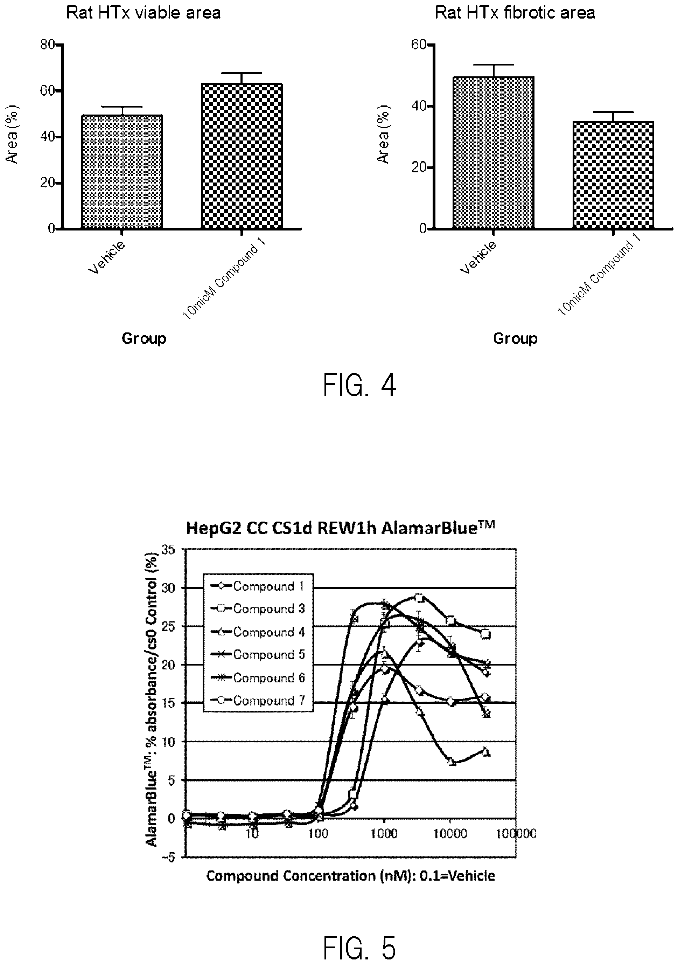

To each of 7 to 8 week-old LEW rats as donors, thoracotomy and laparotomy under general anesthesia were performed to expose the heart, and then, the superior vena cava and the inferior vena cava thereof were ligated. Further, the pulmonary artery and the aorta were cut, and while being perfused through the aortic arch with Vehicle (UW solution at 4°C) or the UW solution to which 10 µM of the compound 1 was added, the pulmonary vein was ligated and cut, so that the heart was removed. The heart was immersed in Vehicle (UW solution at 4°C) or the UW solution to which 10 µM of the compound 1 was added, and was in 24-hour cold storage at 4°C. To each of 7 to 8 week-old LEW rats as recipients, laparotomy under general anesthesia was performed to expose the abdominal aorta and the vena cava. Then, the heart in cold storage was taken out of the UW solution, and ectopic transplantation was carried out, by end-to-side anastomosis between the pulmonary artery and the aorta of the donor and the abdominal vena cava and the aorta of the recipient, respectively, followed by laparotomy closure. The heart was removed one week after the transplantation, and the viable area and fibrotic area of myocardium in the heart were compared by using Masson's trichrome staining (N=5 in each group). Exemplary results thereof are shown in FIG. 4.

As illustrated in FIG. 4, the hearts in the compound 1 administered group had more viable myocardium, and less fibrotic one, as compared with the hearts in the Vehicle group. It can be determined from these results that cold damage can be suppressed by the cold storage under the presence of the compound 1, and the hearts after the 24-hour cold storage were in a state more suitable for transplantation.

[Example 5: Assessment 5 of Cold Damage Suppression Effects]

Using the compounds 1 and 3 to 7 synthesized by the above-described scheme, the cell line HepG2 derived from a human liver cancer was cultured under the presence of the respective compounds at low temperature, and cold damage suppression effects were assessed.

HepG2 cells were cultured at 37°C so as to be at a density of 80 to 90 %, and thereafter, the medium was replaced with a medium (DMEM) at 4°C to which any one of the compounds 1 and 3 to 7 had been added preliminarily. Then, the culturing at 4°C in a low temperature incubator was performed for 24 hours. After the culturing at low temperature for 24 hours, rewarming was carried out by a medium at 37°C, and the viability of cells one hour after the rewarming was after the rewarming was determined by detecting enzyme activity using alamarBlue™ The compound concentration was varied from 0 M (Vehicle) to 50 µM, so that the concentration dependency of the compound was observed. Exemplary results thereof are shown in FIG. 5.

As illustrated in FIG. 5, the low-temperature preservation under the presence of any one of the compounds 1 and 3 to 7 provides the concentration-dependent amelioration of the deterioration of cell viability during the low-temperature preservation, thereby suppressing cold damage.

[Example 6: Assessment 6 of Cold Damage Suppression Effects]

Assessment of cold damage suppression effects was carried out in the same manner as that in Example 1 except for the use of the following compounds 8 to 10.

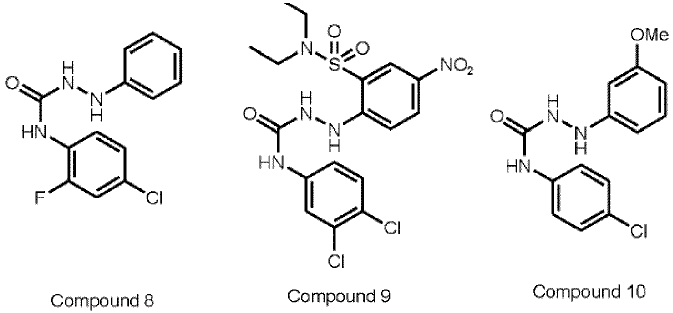

As a result, the low-temperature preservation under the presence of any one of the compounds 8 to 10, with a more or less poor activity as compared with the case of the compound 1, however, provided the concentration-dependent amelioration of the deterioration of cell viability during the low-temperature preservation, thereby suppressing cold damage.

## Claims

1. A method for suppressing damage to a biological material caused by cold storage, the method comprising inhibiting a sphingosine-1-phosphoric acid (S1P) - mediated signal transduction pathway in the biological material.

2. The method according to claim 1, wherein the inhibiting includes bringing the biological material into contact with a substance that inhibits the S1P signal transduction pathway.

3. The method according to claim 2, wherein the substance that inhibits the S1P signal transduction pathway is at least one selected from the group consisting of an S1P receptor (S1PR) inhibiting compound and a sphingosine kinase (SPHK) inhibiting compound.

4. The method according to any one of claims 1 to 3, the method further comprising preserving the biological material at low temperature,
wherein the inhibiting a signal transduction pathway is carried out before the preserving the biological material at low temperature, or simultaneously with the start of the preserving the biological material at low temperature.

5. A method for suppressing damage to a biological material caused by cold storage,
the method comprising bringing the biological material into contact with a compound expressed by Formula (I) or (I') below, or with a pharmaceutically acceptable salt of any of these compounds, 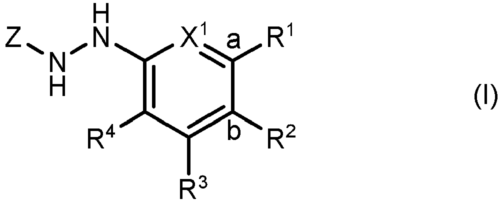 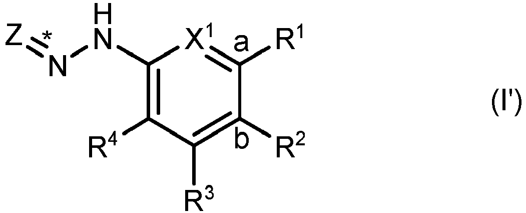
where X¹ represents N or CR⁵,
R¹ and R² independently represent a hydrogen atom; a halogen atom, a C₁₋₈ alkyl group, a C₁₋₈ alkyl group substituted with a halogen atom, a C₁₋₈ alkoxy group, a C₂₋₈ alkynyl group, a C₁₋₈ alkylsulfonyl group, or a cyano group, or alternatively, R¹ and R² form an aromatic ring which may be substituted, or an aromatic heterocycle which may be substituted, together with atoms denoted by "a" and "b",
R³, R⁴, and R⁵ independently represent a hydrogen atom, a halogen atom, a C₁₋₈ alkyl group, a C₁₋₈ alkyl group substituted with a halogen atom, a C₁₋₈ alkoxy group, a C₂₋₈ alkynyl group, a C₁₋₈ alkylsulfonyl group, or a cyano group, and
Z represents: 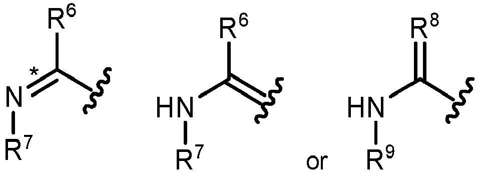
where R⁶ represents -C(=O)R¹⁰ or -CH₂R¹¹,
where R¹⁰ and R¹¹ each represent a C₂₋₈ alkyl group which may be substituted, a phenyl group which may be substituted, a C₂₋₈ alkoxy group which may be substituted, a heterocyclic aromatic group which may be substituted, or -NR¹²R¹³,
where R¹² and R¹³ independently represent a hydrogen atom or a C₁₋₈ alkyl group which may be substituted, or alternatively, R¹² and R¹³ form a nitrogen-containing heterocyclic group which may be substituted, together with an adjacent nitrogen atom,
R⁷ represents a phenyl group which may be substituted, a pyridinyl group which may be substituted, a quinoline group, or an isoquinoline group,
R⁸ represents O or NR¹⁴, where R¹⁴ represents a hydrogen atom, a C₂₋₈ alkyl group which may be substituted, a phenyl group which may be substituted, a C₂₋₈ alkoxy group which may be substituted, or a heterocyclic aromatic group which may be substituted, and
R⁹ represents a phenyl group which may be substituted, a pyridinyl group which may be substituted, a quinoline group, or an isoquinoline group.

6. The method according to claim 5,
wherein the compound expressed by Formula (I) or (I') is a compound expressed by: 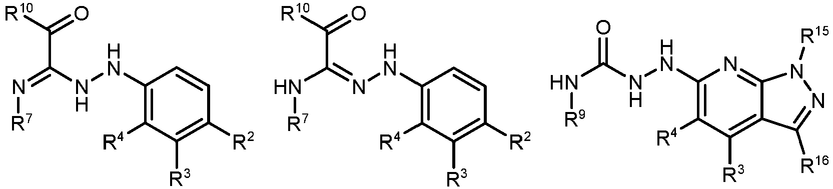 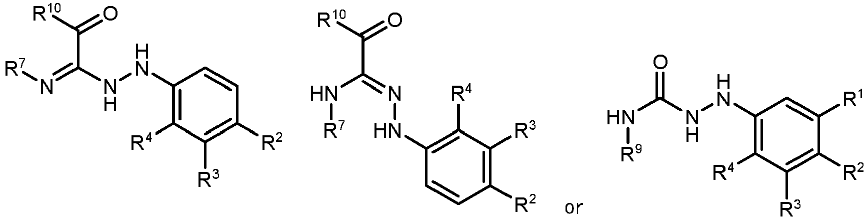
where R¹, R², R³, and R⁴ independently represent a hydrogen atom, a halogen atom, a methyl group, an ethyl group, an isopropyl group, a propyl group, a trifluoromethyl group, a cyano group, a methoxy group, an acetyl group, a methanesulfonyl group, or an ethynyl group,
R⁷ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group,
R⁹ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group,
R¹⁰ represents an n-butyl group, a tert-butyl group, a 1-ethylpropyl group, a 2,2-dimethylpropyl group, a phenyl group which may be substituted, a furyl group which may be substituted, an ethoxy group, or -NR¹²R¹³,
where R¹² and R¹³ independently represent a hydrogen atom or a tert-butyl group, or alternatively, R¹² and R¹³ together with an adjacent nitrogen atom form a piperidine ring, and
R¹⁵ and R¹⁶ independently represent a hydrogen atom, a halogen atom, a methyl group, a trifluoromethyl group, a cyano group, a methoxy group, an acetyl group, a methanesulfonyl group, or an ethynyl group.

7. The method according to any one of claims 1 to 4,
wherein the S1PR inhibiting compound is the compound expressed by Formula (I) or (I') as defined in claim 5 or 6, or a pharmaceutically acceptable salt of the compound.

8. The method according to any one of claims 1 to 7,
wherein the biological material is at least one selected from the group consisting of organs, tissues, cells, body fluids, blood preparations, and cell sheets.

9. A composition for preventing cold storage damage containing, as an active ingredient, a substance that inhibits a sphingosine-1-phosphoric acid (S1P)-mediated signal transduction pathway.

10. A composition for preventing cold storage damage containing, as an active ingredient, a compound expressed by Formula (I) or (I') or a pharmaceutically acceptable salt of any of the compounds, 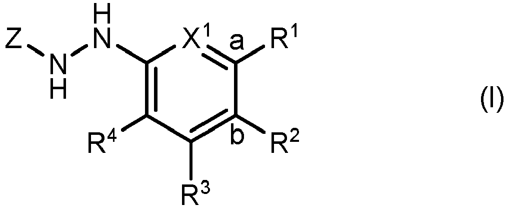 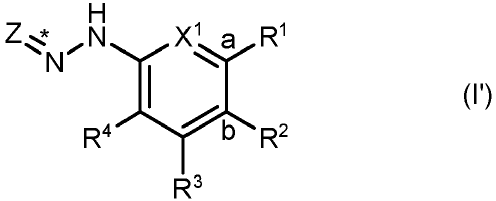
where X¹ represents N or CR⁵,
R¹ and R² independently represent a hydrogen atom; a halogen atom, a C₁₋₈ alkyl group, a C₁₋₈ alkyl group substituted with a halogen atom, a C₁₋₈ alkoxy group, a C₂₋₈ alkynyl group, a C₁₋₈ alkylsulfonyl group, or a cyano group, or alternatively, R¹ and R² form an aromatic ring which may be substituted, or an aromatic heterocycle which may be substituted, together with atoms denoted by "a" and "b",
R³, R⁴, and R⁵ independently represent a hydrogen atom, a halogen atom, a C₁₋₈ alkyl group, a C₁₋₈ alkyl group substituted with a halogen atom, a C₁₋₈ alkoxy group, a C₂₋₈ alkynyl group, a C₁₋₈ alkylsulfonyl group, or a cyano group, and
Z represents: 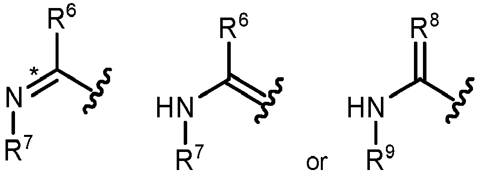
where R⁶ represents -C(=O)R¹⁰ or -CH₂R¹¹,
where R¹⁰ and R¹¹ each represent a C₂₋₈ alkyl group which may be substituted, a phenyl group which may be substituted, a C₂₋₈ alkoxy group which may be substituted, a heterocyclic aromatic group which may be substituted, or -NR¹²R¹³,
where R¹² and R¹³ independently represent a hydrogen atom or a C₁₋₈ alkyl group which may be substituted, or alternatively, R¹² and R¹³ form a nitrogen-containing heterocyclic group which may be substituted, together with an adjacent nitrogen atom,
R⁷ represents a phenyl group which may be substituted, a pyridinyl group which may be substituted, a quinoline group, or an isoquinoline group,
R⁸ represents O or NR¹⁴, where R¹⁴ represents a hydrogen atom, a C₂₋₈ alkyl group which may be substituted, a phenyl group which may be substituted, a C₂₋₈ alkoxy group which may be substituted, or a heterocyclic aromatic group which may be substituted, and
R⁹ represents a phenyl group which may be substituted, a pyridinyl group which may be substituted, a quinoline group, or an isoquinoline group.

11. The composition according to claim 10,
wherein the compound expressed by Formula (I) or (I') is a compound expressed by: 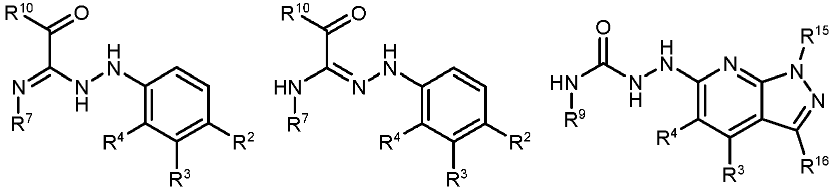 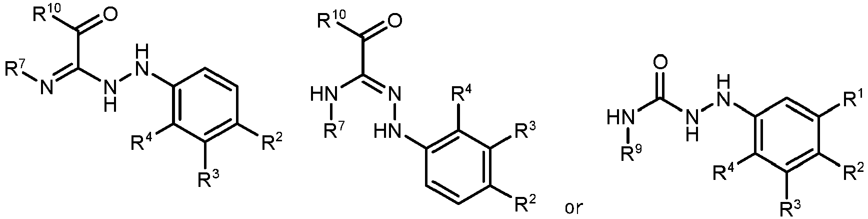
where R¹, R², R³, and R⁴ independently represent a hydrogen atom, a halogen atom, a methyl group, an ethyl group, an isopropyl group, a propyl group, a trifluoromethyl group, a cyano group, a methoxy group, an acetyl group, a methanesulfonyl group, or an ethynyl group,
R⁷ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group,
R⁹ represents a halogen atom; a trifluoromethyl group; a cyano group; a phenyl group substituted with one, two, or more substituent groups selected from the group consisting of methoxy groups and ethynyl groups; a phenyl group; a pyridinyl group substituted with one, two, or more halogen atoms; a pyridinyl group; a quinoline group; or an isoquinoline group,
R¹⁰ represents an n-butyl group, a tert-butyl group, a 1-ethylpropyl group, a 2,2-dimethylpropyl group, a phenyl group which may be substituted, a furyl group which may be substituted, an ethoxy group, or -NR¹²R¹³.
where R¹² and R¹³ independently represent a hydrogen atom or a tert-butyl group, or alternatively, R¹² and R¹³ together with an adjacent nitrogen atom form a piperidine ring, and
R¹⁵ and R¹⁶ independently represent a hydrogen atom, a halogen atom, a methyl group, a trifluoromethyl group, a cyano group, a methoxy group, an acetyl group, a methanesulfonyl group, or an ethynyl group.

12. The composition according to claim 9, wherein the substance that inhibits an S1P-mediated signal transduction pathway is the compound expressed by Formula (I) or (I') as defined in claim 10 or 11, or a pharmaceutically acceptable salt of the compound.

13. A method for preserving a biological material at low temperature, the method comprising inhibiting a sphingosine-1-phosphoric acid (S1P)-mediated signal transduction pathway in the biological material.

14. A solution for preserving a biological material, the solution containing, as an active ingredient, a substance that inhibits a sphingosine-1-phosphoric acid (S1P)-mediated signal transduction pathway.

15. A solution for preserving a biological material, the solution containing, as an active ingredient, the compound expressed by Formula (I) or (I') as defined in claim 10 or 11, or a pharmaceutically acceptable salt of the compound.
